# EUROPÄISCHE PATENTSCHRIFT

(11) **EP 3 061 474 B1**
(45) Veröffentlichungstag und Bekanntmachung des Hinweises auf die Patenterteilung: **17.04.2019**
(21) Anmeldenummer: 16155926.5
(22) Anmeldetag: 16.02.2016
(51) Int. Cl.: A61M 1/10

(54) **GEHÄUSEFRONTINTEGRIERTE BLUTPUMPE**
BLOOD PUMP INTEGRATED INTO THE FRONT OF A HOUSING
POMPE A SANG ENCASTREE EN FAÇADE D'UN BOITIER

(30) Priorität: 25.02.2015 DE 102015102658
(43) Veröffentlichungstag der Anmeldung: 31.08.2016
(73) Patentinhaber: B. Braun Avitum AG, 34212 Melsungen (DE)
(72) Erfinder: SCHÄFER, Oliver, 36286 Neuenstein (DE); RITTER, Kai-Uwe, 91126 Rednitzhembach (DE)
(74) Vertreter: Winter, Brandl, Fürniss, Hübner, Röss, Kaiser, Polte - Partnerschaft mbB

(56) Entgegenhaltungen:
- WO-A1-90/06781
- WO-A2-97/10436
- DE-T2- 69 722 065
- US-A- 4 734 184
- US-A- 6 071 095

## Beschreibung

Die Erfindung betrifft eine Vorrichtung zur extrakorporalen Blutbehandlung gemäß dem Oberbegriff des Anspruchs 1, genauer gesagt eine Vorrichtung zur extrakorporalen Blutbehandlung, insbesondere Dialysemaschine, mit einer Peristaltikpumpe zum Fördern von Fluid von einer Niederdruckseite zu einer Hochdruckseite, welche Peristaltikpumpe einen um eine Achse rotierbaren Rotor und eine um die Achse bogenförmig ausgebildete Stützfläche aufweist, wobei eine elastisch verformbare Fluidleitung zwischen dem Rotor und der Stützfläche positionierbar ist und bei Rotation des Rotors eine Querschnittsverengung ausbildend zwischen diesem und der Stützfläche verformt wird, so dass bei gegenüber der Stützfläche rotierendem Rotor Fluid in der Fluidleitung von der Niederdruckseite zur Hochdruckseite gefördert wird, wobei die Vorrichtung ein als Blechformteil ausgebildetes Maschinengehäuse aufweist.

Bekannte Peristaltikpumpen bei Medizingeräten zur extrakorporalen Blutbehandlung bestehen in der Regel aus einem Rotor, einem Pumpengehäuse und einer zwischen Rotor und Pumpengehäuse angeordneten elastischen Schlauchleitung als Fluidleitung. Das Pumpengehäuse bildet eine Stützfläche für die Fluidleitung aus. Aus dem Stand der Technik sind aufgesetzte Blutpumpengehäuse bekannt. Zum Beispiel ist ein Pumpengehäuse bekannter Peristaltikpumpen für derartige Medizingeräte als separates Aluminium-Frästeil oder Kunststoff-Spritzgussteil ausgebildet und auf eine Gehäusefront des Gerätes montiert. Die Realisierung des Pumpengehäuses mittels eines separaten Bauteils ist aufgrund von relativ hohen Produktions- und Lagerkosten infolge des zusätzlichen Bauteils von Nachteil. Des Weiteren ist ein Montagevorgang zur Implementierung des Pumpengehäuses an das Maschinengehäuse erforderlich, was zeit- und kostenintensiv ist. Schließlich sind derartig komplex geformte Frästeile an sich kostenintensiv. Die Verwendung eines Pumpengehäuses aus Kunststoff kann hier zwar teilweise Abhilfe schaffen, allerdings auf Kosten von Festigkeit und Verschleißbeständigkeit. Die DE 697 22 065 T2 und die US 6 071 095 A sind als nächstliegender Stand der Technik anzusehen.

Ausgehend von dem vorstehend beschriebenen Stand der Technik liegt der vorliegenden Erfindung die Aufgabe zugrunde, die zuvor angeführten Nachteile zu beseitigen, insbesondere eine Vorrichtung zur extrakorporalen Blutbehandlung zu schaffen, die fertigungs-, montage- und kostenoptimiert herstellbar und verschleißbeständig ist.

Diese Aufgabe wird gelöst durch die Merkmale des Anspruchs 1.

Eine erfindungsgemäße Vorrichtung zur extrakorporalen Blutbehandlung, insbesondere Dialysemaschine, weist eine Peristaltikpumpe zum Fördern von Fluid von einer Niederdruckseite zu einer Hochdruckseite mit einem um eine Rotorachse rotierbaren Rotor und einer um die Rotorachse bogenförmig ausgebildete Stützfläche auf, wobei eine elastisch verformbare Fluidleitung zwischen dem Rotor und der Stützfläche positionierbar ist und bei Rotation des Rotors eine Querschnittsverengung ausbildend zwischen diesem und der Stützfläche verformt wird, so dass bei gegenüber der Stützfläche rotierendem Rotor Fluid in der Fluidleitung von der Niederdruckseite zur Hochdruckseite gefördert wird, wobei die Vorrichtung ein als Blechformteil ausgebildetes Maschinengehäuse aufweist, wobei die Stützfläche durch Umformen in dem Maschinengehäuse bzw. einem Maschinengehäuseelement oder -teil ausgebildet ist, insbesondere einteilig mit dem Maschinengehäuse bzw. dem Maschinengehäuseelement oder -teil ausgebildet ist.

Nach der Erfindung ist die Stützfläche, die man auch als Lauffläche bezeichnen kann, in das Maschinengehäuse integriert, insbesondere einteilig mit dem Maschinengehäuse oder zumindest einem Blechformteils des Maschinengehäuses, zum Beispiel einer Maschinenfront, ausgebildet. Infolge dessen ist die Anzahl der Einzelteile, die bei der Montage der Blutbehandlungsvorrichtung zu montieren, zu lagern und zu verwalten sind, in vorteilhafter Weise verhältnismäßig gering, was Montage und Organisation vereinfacht und Kosten minimiert. Des Weiteren ist die Stützfläche besonders stabil und fest, zum einen aufgrund der einteiligen Ausführung mit dem Gehäuse, zum anderen aufgrund einer üblicherweise mit einer Umformung einhergehenden Materialverfestigung, was Verschleiß minimiert. Die Stützfläche ist insbesondere steifer als eine herkömmliche Kunststoffstützfläche. Der bei der Herstellung des Maschinengehäuses vorliegende fertigungstechnische Aufwand wird durch das Ausbilden der Stützfläche nicht wesentlich gesteigert, da dieses zur Aufnahme weiterer Funktionskomponenten, wie zum Beispiel Schalter, Anzeigen, elektrische oder hydraulische Anschlüsse, eine Antriebseinheit für den Rotor, ein Deckel zum Verschließen der Pumpe nach Einlegen der elastischen Fluidleitung, etc. hergerichtet werden kann, zum Beispiel durch Umformen, Stanzen, Bohren etc. Zusammengefasst ausgedrückt liegt ein mit der Erfindung erzielter Vorteil in einer hohen Funktionsintegration und einer daraus resultierende Kostensenkung der Maschine. Schließlich kann elektrostatische Aufladung, die beim Betrieb herkömmlicher Peristaltikpumpen, insbesondere solchen mit einer Kunststoffstützfläche oder einer nicht einteilig mit dem Maschinengehäuse ausgebildeten Metallstützfläche, in nachteiliger Weise auftreten kann, minimiert werden.

Die Peristaltikpumpe der erfindungsgemäßen Vorrichtung fördert ein definiertes Volumen eines Mediums, wie zum Beispiel Blut oder Dialysefluid, von der Niederdruckseite, in der Regel die arterielle Seite, auf die Hochdruckseite, in der Regel die venöse Seite. Die elastische Fluidleitung wird in diese zwischen Rotor und Stützfläche schlaufenförmig eingelegt. Der Rotor und die die elastische Fluidleitung stützende Stützfläche sind derart ausgebildet und aufeinander abgestimmt, dass zwischen ihnen eine Förderstrecke ausgebildet ist. In deren Verlauf kommt es bei Rotation des Rotors um die Rotorachse zur Verformung und Abklemmung der elastisch verformbaren Fluidleitung. Der Rotor ist derart ausgebildet, dass die Fluidleitung nur punktuell oder abschnittsweise zusammengequetscht wird. Er kann zum Beispiel gegen die Fluidleitung vorgespannte und/oder gegenüber der Rotorachse relativpositionierbare Quetschelemente aufweisen. Die durch den Kontakt mit dem Rotor verursachte Quetschstelle wandert mit dem rotierenden Rotor mit und wird quasi durch die Fluidleitung von der Niederdruckseite zur Hochdruckseite bewegt. Infolge dessen wird Fluid in Förderrichtung aus der Fluidleitung herausgedrückt. Nachfließendes Fluid wird durch Niederdruck, insbesondere Vakuum, der aufgrund elastischer Rückformung der Fluidleitung nach der Verformung durch den Rotor entsteht, in die Leitung hineingezogen. Die elastisch verformbare Fluidleitung kann zum Beispiel ein Schlauch sein.

Im Bereich der Förderstrecke wird die Fluidleitung in der vorstehend beschriebenen Weise verformt und bei ordnungsgemäßer Funktion im Wesentlichen fluiddicht zusammengequetscht. Die Quetschelemente können unmittelbar am Rotor ausgebildet sein, insbesondere einstückig mit dem Rotor. Sie können alternativ auf Rotorarmen angeordnet sein. Die Quetschelemente können insbesondere als Quetschrollen oder Andruckrollen, die in vorteilhafter Weise materialschonend an der Fluidleitung abrollen, oder Gleitschuhe, die sich gleitend über die Fluidleitung bewegen, ausgebildet sein. Die Quetschelemente können insbesondere in radialer Richtung positionierbar und radial nach außen, also in eine die Fluidleitung zusammenquetschende Position vorgespannt sein. Vorzugsweise erfolgt diese Vorspannung mittels Federelementen.

Mit der Erfindung können insbesondere die folgenden Vorteile erzielt werden:
- höhere Funktionsintegration des Maschinengehäuses, z.B. einer Fronttür,
- daraus resultierende Kostensenkung der Maschine,
- bessere Steifigkeit der Stützfläche gegenüber einer in Kunststoff integrierten Stützfläche,
- gegenüber dem Stand der Technik verbessertes Verhalten hinsichtlich elektrostatischer Entladung bzw. ESD (Electrostatic Discharge).

Vorteilhafte Ausführungsformen der Erfindung sind in den Unteransprüchen beansprucht und werden nachfolgend näher erläutert.

Nach einer Ausführungsform kann die Stützfläche durch Kaltumformen, insbesondere durch Tiefziehen, in dem Maschinengehäuse, insbesondere in einem eine Maschinenfront ausbildenden Blechteil, ausgebildet sein. Die Ausbildung der Stützfläche kann so ohne Aufwand in einen üblichen Prozess zur Herstellung des Maschinengehäuses oder zumindest von Teilen davon integriert werden.

Nach einer weiteren Ausführungsform der Erfindung kann die Stützfläche randseitig einer in das Maschinengehäuse eingebrachten Vertiefung ausgebildet sein. Dabei ist von Vorteil, dass die Fluidpumpe, die später in dieser Vertiefung angeordnet ist, zumindest teilweise, vorzugsweise vollständig, in die Maschinenfront integriert und/oder versenkt ist und so gehaust und geschützt ist. Durch den versenkten Rotor wird die Sicherheit des Benutzers erhöht bzw. die Pumpe, insbesondere der Rotor besser vor äußeren Einwirkungen geschützt.

Alternativ kann die Stützfläche randseitig einer in das Maschinengehäuse eingebrachten Erhebung ausgebildet sein. Zum Beispiel kann ein die Stützfläche aufweisender Wulst aus der Blechebene des Gehäuses vorstehen, was eine einfache Anordnung der elastischen Fluidleitung ermöglicht.

Vorzugsweise kann die Stützfläche teilzylinderförmig ausgebildet sein. Insbesondere kann die Stützfläche gegenüber der Blechebene des Maschinengehäuses um einen Winkel α geneigt ausgebildet sein, wobei der Winkel α in einem Bereich zwischen ca. 120° und ca. 95°, vorzugsweise zwischen ca. 115° und ca. 100°, besonders bevorzugt zwischen ca. 110° und ca. 105° liegen kann.

Nach einer Ausführungsform der Erfindung kann die Stützfläche einen Bodenabschnitt oder eine Bodenfläche umgeben, der bzw. die radial innerhalb der Stützfläche ausgebildet ist, insbesondere zusammen mit der Stützfläche umgeformt ist. Nach einer Ausführungsform der Erfindung kann der Bodenabschnitt bzw. die Bodenfläche eine Axiallagerfläche für die Fluidleitung und/oder den Rotor ausbilden. Der Bodenabschnitt kann gegenüber der Stützfläche im Wesentlichen zumindest teilweise orthogonal ausgebildet sein. Bei der vorstehend beschriebenen Ausführungsform kann die elastische Fluidleitung besonders einfach und ohne Knicke oder starke Richtungsänderungen am Maschinengehäuse entlang geführt sein und in die Vertiefung mit der Stützfläche eingelegt werden. Eine Fluidströmung durch die Fluidleitung wird dabei nur möglichst sanften und geringen Richtungswechseln unterzogen, was Strömungswiderstände in der Leitung gering hält.

Die Rotorachse ist vorzugsweise parallel zur Stützebene ausgebildet und orientiert. Auf diese Weise wird ein möglichst vollständiges Zusammenquetschen der elastischen Fluidleitung sichergestellt.

Die Vertiefung weist vorzugsweise eine im Wesentlichen hufeisenförmige Außenkontur auf, wobei beidseitig der Stützfläche Einlaufflächen ausgebildet sind, die zur Rotorachse vorzugsweise parallel ausgebildet sind. Im Bereich dieser Einlaufflächen kommt es in vorteilhafter Weise zu einer langsamen Verformung der Fluidleitung durch den Rotor, was besonders materialschonend ist.

Man kann auch sagen, dass die Erfindung eine Vorrichtung zur extrakorporalen Blutbehandlung betrifft, mit einer in eine Blechgehäusefront integrierten Stütz- oder Lauffläche. Diese ist Bestandteil einer Peristaltikpumpe, insbesondere Blutpumpe, beispielsweise einer peristaltisch arbeitenden Rollenpumpe oder Schlauchpumpe für die Medizintechnik. Ein Rotor ermöglicht zusammen mit den elastischen Materialeigenschaften des Pumpensegmentes eines Überleitsystems eine Pumpfunktion, die eine Fluidförderung, insbesondere Blutförderung zu einem Dialysator sicherzustellen. Dabei wird das Pumpensegment des Überleitsystems schlaufenförmig gegen die in der Blechgehäusefront integrierte zylindrische Stütz- oder Lauffläche eingelegt. Die Stütz- oder Lauffläche nimmt dabei mit dem zylindrischen Durchmesser und dem zylindrischen Umschlingungswinkel Einfluss auf die Menge des geförderten Mediums.

Ein weiterer Aspekt der Erfindung betrifft ein Gehäuseteil für eine Vorrichtung zur extrakorporalen Blutbehandlung, insbesondere Dialysemaschine, insbesondere nach einem der vorstehenden Ansprüche, wobei das Gehäuseteil aus einem Blech geformt ist. Dabei ist in dem Gehäuseteil durch Umformen eine Vertiefung ausgebildet, welche zur Aufnahme eines um eine Rotorachse rotierbaren Rotors und eines elastisch verformbaren Fluidleitungsabschnitts einer Peristaltikpumpe dient, wobei ein um die Rotorachse bogenförmig ausgebildeter Rand der Vertiefung eine Stützfläche bildet, gegen welche der Fluidleitungsabschnitt mittels des Rotors drückbar ist. Auf diese Weise bildet ein Gehäuseteil, z.B. eine Gehäuseblechwandung bzw. ein Gehäuseblechwandungsabschnitt einen Teil der Peristaltikpumpe.

Ein weiterer Aspekt der Erfindung betrifft ein Verfahren zur Herstellung eines Gehäuseteils für eine Vorrichtung zur extrakorporalen Blutbehandlung, insbesondere Dialysemaschine, insbesondere nach einem der vorstehenden Aspekte, mit den Schritten Formen des Gehäuseteils aus einem Blech und Ausbilden einer Vertiefung in dem Gehäuseteil durch Umformen, welche zur Aufnahme eines um eine Rotorachse rotierbaren Rotors und eines elastisch verformbaren Fluidleitungsabschnitts einer Peristaltikpumpe dient, wobei ein um die Rotorachse bogenförmig ausgebildeter Rand der Vertiefung eine Stützfläche bildet, gegen welche der Fluidleitungsabschnitt mittels des Rotors zur Querschnittsverengung drückbar ist.

Die Erfindung wird im Folgenden anhand beispielhafter, nicht einschränkender und in den angehängten Figuren gezeigter Ausführungsformen näher erläutert. Dabei zeigt:
Figur 1 eine schematische Darstellung eines Ausschnitts einer Vorrichtung zur extrakorporalen Blutbehandlung,
Figur 2 eine schematische Darstellung eine Pumpengehäuses nach dem Stand der Technik,
Figur 3 eine schematische Darstellung eines in ein Maschinengehäuse integrierten Pumpengehäuses gemäß der Erfindung in einer ersten perspektivischen Ansicht,
Figur 4 das in das Maschinengehäuse integrierte Pumpengehäuses der Figur 3 in einer anderen perspektivischen Ansicht, und
Figur 5 das in das Maschinengehäuse integrierte Pumpengehäuse der Figuren 3 und 4 in einer Schnittansicht.

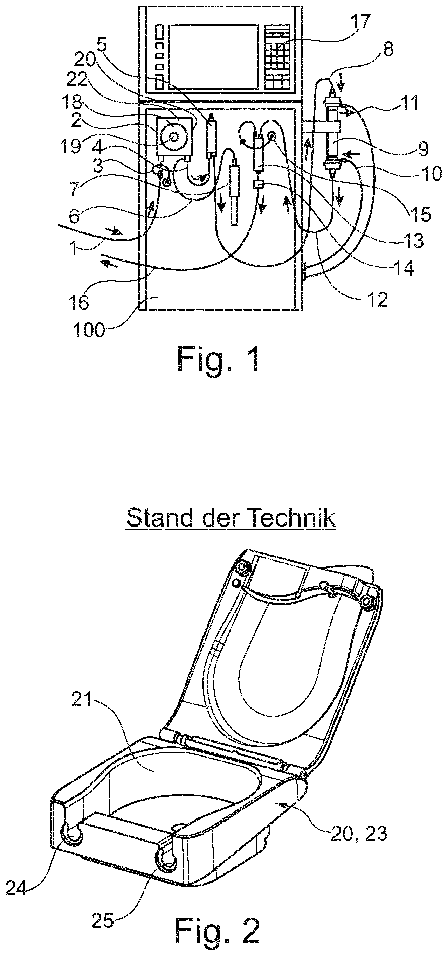

Figur 1 zeigt beispielhaft einen Ausschnitt einer Vorrichtung zur extrakorporalen Blutbehandlung nach der Erfindung. Gezeigt ist im Wesentlichen der gesamte extrakorporale Blutkreislauf der Vorrichtung. Dieser weist eine arterielle Blutleitung 1 auf, mittels der Blut von einem nicht gezeigten Patienten zu einer Peristaltikpumpe 2 der Behandlungsvorrichtung geführt wird. Vor der Peristaltikpumpe 2 ist ein arterieller Druckabnehmer 3 vorgesehen, mit dem der Druck vor der Peristaltikpumpe 2, also der niederdruckseitige Druck gemessen wird. Hochdruckseitig der Peristaltikpumpe 2 führt eine Hochdruckblutleitung 4 zu einem arteriellen Luftfänger 5. Unmittelbar am Ausgang der Peristaltikpumpe 2 kann mittels einer Zuleitung 6 und einer Pumpe 7 Zusatzstoff, z.B. Heparin zur Blutverdünnung, in das im System befindliche Blut gegeben werden.

Vom arteriellen Luftfänger 5 führt eine Leitung 8 unter Hochdruck stehendes aber noch unbehandeltes Blut zu einem Dialysator 9. Diesem wird eingangsseitig Dialysierflüssigkeit über eine Dialysierflüssigkeitszuleitung 10 zugeführt. Im Dialysator 9 wird Blut in bekannter Weise mittels der Dialysierflüssigkeit behandelt, z.B. gereinigt. Verbrauchte Dialysierflüssigkeit wird über eine Dialysierflüssigkeitsableitung 11 vom Dialysator 9 entfernt und einer nicht dargestellten Entsorgung oder Aufbereitung zugeführt. Behandeltes Blut wird mittels einer Blutableitung 12 vom Dialysator 9 zu einem venösen Luftfänger 13 geleitet, wo mittels einer Luftfalle 14 Luft abgeschieden wird. Am venösen Luftfänger 13 ist ein venöser Druckaufnehmer 15 vorgesehen, mit dem der venöse Druck, also der hochdruckseitige Druck, erfasst wird. Von der Luftfalle 14 wird behandeltes Blut über eine venöse Blutleitung 16 zurück zum Patienten geleitet. Dargestellt in Figur 1 ist auch eine Einheit 17 zur Überwachung und Steuerung der Vorrichtung. Die Vorrichtung zur extrakorporalen Blutbehandlung ist mit einem Gehäuse 100 gekapselt, das zumindest teilweise als Blechformteil ausgebildet ist.

Die Peristaltikpumpe 2 weist einen Rotor 18 auf, der um eine Rotorachse 19 rotiert. Die Peristaltikpumpe 2 weist des Weiteren ein in Figur 1 nur schematisch angedeutetes Blutpumpengehäuse 20 mit einer Stützfläche 21 für eine elastisch verformbare Fluidleitung 22 auf. Diese ist zwischen der Stützfläche 21 des Blutpumpengehäuses 20 und Rotor 18 angeordnet und wird bei Rotation des Rotors 18 deformiert. Die Fluidleitung 22 ist eingangsseitig, also niederdruckseitig, mit der arteriellen Blutleitung 1 und ausgangsseitig, also hochdruckseitig, mit der Blutleitung 4 verbunden. Sie wird zwischen dem Rotor 18 und der Stützfläche 21 derart deformiert, dass ihr Querschnitt im fehlerfreien Normalbetrieb der Pumpe 2 vorzugsweise vollständig zusammengequetscht, also im Wesentlichen fluiddicht geschlossen wird.

Figur 2 zeigt ein Blutpumpengehäuse 20 nach dem Stand der Technik. Dieses ist als separates Aluminium-Frästeil 23 ausgebildet, das auf die Gehäusefront 100 des Gerätes montiert wird. Das Aluminium-Frästeil 23 ist verhältnismäßig komplex mit einer Einlassnut 24 und einer Auslassnut 25 für die Fluidleitung 22. Die Stützfläche 21 ist im Aluminium-Frästeil 23 durch eine eingefräste Vertiefung ausgebildet, was einen hohen Materialverbrauch und Fertigungsaufwand zur Folge hat.

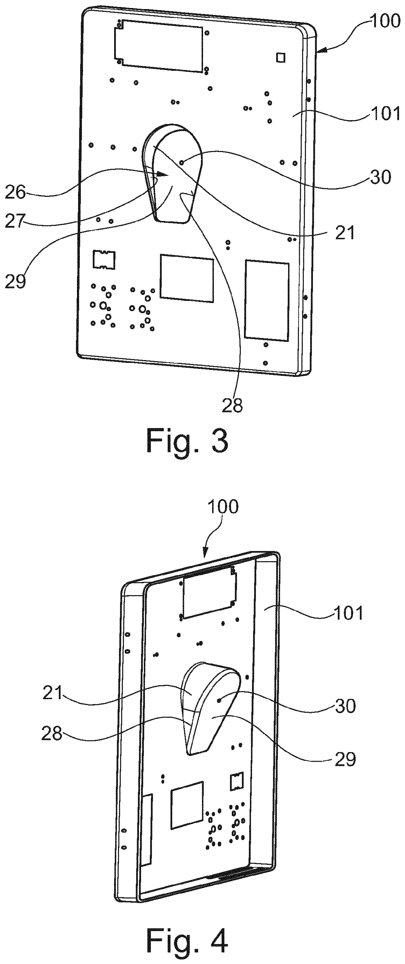

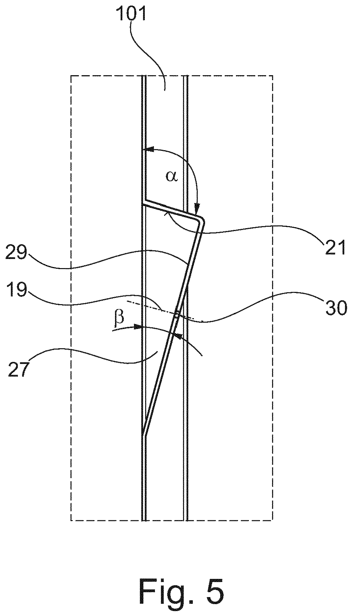

Die Figuren 3, 4 und 5 zeigen ein gemäß der Erfindung in ein Blech 101 der Gehäusefront 100 integriertes Blutpumpengehäuse 20. Dessen Stützfläche 21 ist ausgebildet, indem mittels eines geeigneten Umformverfahrens, zum Beispiel mittels Tiefziehen, eine Vertiefung 26 direkt in die Blechfront 101 des Gerätegehäuses 100 eingebracht ist. Die Vertiefung 26 ist durch die Stützfläche 21, eine einlaufseitige Wand 27, eine auslaufseitige Wand 28 und eine Bodenwand 29 begrenzt. Bei der in den Figuren dargestellten Ausführungsform ist die Vertiefung 26 gegenüber der übrigen Gehäusefront geneigt ausgebildet (siehe insbesondere Figur 5). Daher ist die Stützfläche 21 um einen Winkel α und die Bodenwand 29 um einen Winkel β gegenüber der übrigen Gehäusefront geneigt. Dies ist insbesondere in Figur 5 gut zu erkennen. Diese geneigte Anordnung dient dem Zweck, eine Zuführung der Fluidleitung 22 in das Pumpengehäuse 20 hinein und aus diesem heraus ohne oder nur unter möglichst geringem Knicken der Fluidleitung 22 zu ermöglichen.

Die Stützfläche 21 ist in Form eines Teil(kreis-)zylinders ausgebildet. Dessen Mittelachse fällt mit der Rotorachse 19 zusammen und ist gegenüber der Gehäusefront um den Winkel α geneigt. Zur Durchführung der Rotorachse 19 durch das Gehäuse 100 ist in der Bodenwand 29 eine Achsaufnahme 30 vorgesehen.

Weiterhin können weitere Komponenten, die zum Betrieb der Pumpe notwendig sind, wie zum Beispiel in den Figuren nicht dargestellte Deckel, Führungselemente für das Pumpensegment des Überleitsystems und eine Antriebseinheit direkt an der Blechfront montiert sein bzw. werden.

Wie bereits oben angedeutet wird die Gehäusefront 100 aus einem Blech geformt und darin durch Umformen die Vertiefung 26 ausgebildet, welche zur Aufnahme des um die Rotorachse 19 rotierbaren Rotors 18 und des elastisch verformbaren Fluidleitungsabschnitts 22 der Peristaltikpumpe 2 dient, wobei der um die Rotorachse 19 bogenförmig ausgebildeter Rand der Vertiefung 26 die Stützfläche 21 bildet, gegen welche der Fluidleitungsabschnitt 22 mittels des Rotors 19 zur Querschnittsverengung drückbar ist.

## Patentansprüche

1. Vorrichtung zur extrakorporalen Blutbehandlung, insbesondere Dialysemaschine, mit einer Peristaltikpumpe (2) zum Fördern von Fluid von einer Niederdruckseite zu einer Hochdruckseite, welche Peristaltikpumpe (2) einen um eine Rotorachse (19) rotierbaren Rotor (18) und eine um die Rotorachse (19) bogenförmig ausgebildete Stützfläche (21) aufweist, wobei eine elastisch verformbare Fluidleitung (22) zwischen dem Rotor (18) und der Stützfläche (21) positionierbar ist und bei Rotation des Rotors (18) eine Querschnittsverengung ausbildend zwischen diesem und der Stützfläche (21) verformt wird, so dass bei gegenüber der Stützfläche (21) rotierendem Rotor (18) Fluid in der Fluidleitung (22) von der Niederdruckseite zur Hochdruckseite gefördert wird,
wobei die Vorrichtung ein als Blechformteil (101) ausgebildetes Maschinengehäuseteil (100) aufweist,
**dadurch gekennzeichnet, dass** die Stützfläche (21) durch Umformen in dem Maschinengehäuseteil (100, 101) ausgebildet ist.

2. Vorrichtung zur extrakorporalen Blutbehandlung nach Anspruch 1, wobei die Stützfläche (21) durch Kaltumformen, insbesondere durch Tiefziehen ausgebildet ist.

3. Vorrichtung zur extrakorporalen Blutbehandlung nach Anspruch 1 oder 2, wobei die Stützfläche (21) randseitig einer in das Maschinengehäuseteil (100, 101) eingebrachten Vertiefung (26) oder Erhebung ausgebildet ist.

4. Vorrichtung zur extrakorporalen Blutbehandlung nach einem der vorstehenden Ansprüche, wobei die Stützfläche (21) teilzylinderförmig ausgebildet ist.

5. Vorrichtung zur extrakorporalen Blutbehandlung nach einem der vorstehenden Ansprüche, wobei die Stützfläche (21) gegenüber der Blechebene des Maschinengehäuseteils (100, 101) um einen Winkel α geneigt ausgebildet ist, wobei der Winkel α in einem Bereich zwischen ca. 120° und ca. 95°, vorzugsweise zwischen ca. 115° und ca. 100°, besonders bevorzugt zwischen ca. 110° und ca. 105° liegt.

6. Vorrichtung zur extrakorporalen Blutbehandlung nach einem der vorstehenden Ansprüche, wobei die Stützfläche (21) einen Bodenabschnitt oder ein Bodenfläche (29) umgibt, der radial innerhalb der Stützfläche (21) ausgebildet ist, insbesondere zusammen mit der Stützfläche (21) umgeformt ist.

7. Vorrichtung zur extrakorporalen Blutbehandlung nach Anspruch 6, wobei der Bodenabschnitt oder die Bodenfläche (29) eine Axiallagerfläche für die Fluidleitung (22) und/oder den Rotor (18) ausbildet.

8. Vorrichtung zur extrakorporalen Blutbehandlung nach einem der vorstehenden Ansprüche, wobei die Rotorachse (19) parallel zur Stützfläche (21) ist.

9. Vorrichtung zur extrakorporalen Blutbehandlung nach einem der vorstehenden Ansprüche 3 bis 8, wobei die Vertiefung (26) eine im Wesentlichen hufeisenförmige Außenkontur aufweist, wobei beidseitig der Stützfläche (21) Einlaufflächen bzw. Auslaufflächen (27, 28) ausgebildet sind, die zur Rotorachse (19) vorzugsweise parallel ausgebildet sind.

10. Gehäuseteil (100, 101) für eine Vorrichtung zur extrakorporalen Blutbehandlung, insbesondere Dialysemaschine, insbesondere nach einem der vorstehenden Ansprüche, wobei das Gehäuseteil (100, 101) aus einem Blech geformt ist,
**dadurch gekennzeichnet, dass**
in dem Gehäuseteil (100, 101) durch Umformen eine Vertiefung (26) ausgebildet ist, welche zur Aufnahme eines um eine Rotorachse (19) rotierbaren Rotors (18) und eines elastisch verformbaren Fluidleitungsabschnitts (22) einer Peristaltikpumpe (2) dient, wobei ein um die Rotorachse (19) bogenförmig ausgebildeter Rand der Vertiefung eine Stützfläche (21) bildet, gegen welche der Fluidleitungsabschnitt (22) mittels des Rotors zur Querschnittsverengung drückbar ist.

11. Verfahren zur Herstellung eines Gehäuseteils (100, 101) für eine Vorrichtung zur extrakorporalen Blutbehandlung, insbesondere Dialysemaschine, insbesondere nach einem der Ansprüche 1 bis 10, mit den Schritten:
Formen des Gehäuseteils (100, 101) aus einem Blech; und
Ausbilden einer Vertiefung (26) in dem Gehäuseteil (100, 101) durch Umformen, welche zur Aufnahme eines um eine Rotorachse (19) rotierbaren Rotors (18) und eines elastisch verformbaren Fluidleitungsabschnitts (22) einer Peristaltikpumpe (2) dient, wobei ein um die Rotorachse (19) bogenförmig ausgebildeter Rand der Vertiefung eine Stützfläche (21) bildet, gegen welche der Fluidleitungsabschnitt (22) mittels des Rotors zur Querschnittsverengung drückbar ist.

## Claims

1. Device for extracorporeal blood treatment, in particular a dialysis machine, comprising a peristaltic pump (2) for conveying fluid from a low-pressure side to a high-pressure side, said peristaltic pump (2) comprising a rotor (18) which is rotatable around a rotor axis (19) and a support area (21) which is formed around the rotor axis (19) in an arcuate manner, wherein an elastically deformable fluid line (22) can be positioned between the rotor (18) and the support area (21) and is deformed between the rotor and the support area (21) during rotation of the rotor (18), forming a narrowing of the cross-section, so that as the rotor (18) rotates relative to the support area (21) a fluid in the fluid line (22) is conveyed from the low-pressure side to the high-pressure side,
wherein the device comprises a machine housing part (100) designed as a formed sheet metal part (101),
the support area (21) is formed in the machine housing part (100, 101) by plastic deformation.

2. Device for extracorporeal blood treatment according to claim 1, wherein the support area (21) is formed by cold working, in particular by deep drawing.

3. Device for extracorporeal blood treatment according to claim 1 or 2, wherein the support area (21) is formed at the edge of a depression (26) or elevation incorporated in the machine housing part (100, 101).

4. Device for extracorporeal blood treatment according to any one of the preceding claims, wherein the support area (21) is partially cylindrical.

5. Device for extracorporeal blood treatment according to any one of the preceding claims, wherein the support area (21) is inclined by an angle α with respect to the sheet plane of the machine housing part (100, 101), wherein the angle α is in a range between approximately 120° and approximately 95°, preferably between approximately 115° and approximately 100°, particularly preferably between approximately 110° and approximately 105°.

6. Device for extracorporeal blood treatment according to any one of the preceding claims, wherein the support area (21) surrounds a base portion or base area (29) which is formed radially within the support area (21) and is deformed together with the support area (21).

7. Device for extracorporeal blood treatment according to claim 6, wherein the base portion or the base area (29) forms an axial bearing surface for the fluid line (22) and/or the rotor (18).

8. Device for extracorporeal blood treatment according to any one of the preceding claims, wherein the rotor axis (19) is parallel to the support area (21).

9. Device for extracorporeal blood treatment according to any one of the preceding claims 3 to 8, wherein the depression (26) has a substantially horseshoe-shaped outer contour, wherein inlet faces or outlet faces (27, 28) which are preferably parallel to the rotor axis (19) are formed on both sides of the support area (21).

10. Housing part (100, 101) for a device for extracorporeal blood treatment, in particular a dialysis machine, in particular according to any one of the preceding claims, wherein the housing part (100, 101) is formed from sheet metal,
**characterised in that**
a depression (26) is formed in the housing part (100, 101) by plastic deformation and serves to receive a rotor (18) which is rotatable around a rotor axis (19) and an elastically deformable fluid line section (22) of a peristaltic pump (2), wherein an edge of the depression formed around the rotor axis (19) in an arcuate manner forms a support area (21) against which the fluid line section (22) can be pressed by means of the rotor for narrowing of the cross-section.

11. Method for producing a housing part (100, 101) for a device for extracorporeal blood treatment, in particular a dialysis machine, according to any one of claims 1 to 10, comprising the steps of:
forming the housing part (100, 101) from sheet metal; and
forming a depression (26) in the housing part (100, 101) by plastic deformation, the depression serving to receive a rotor (18) which is rotatable around a rotor axis (19) and an elastically deformable fluid line section (22) of a peristaltic pump (2), wherein an edge of the depression formed around the rotor axis (19) in an arcuate manner forms a support area (21) against which the fluid line section (22) can be pressed by means of the rotor for narrowing of the cross-section.

## Revendications

1. Dispositif de traitement extracorporel du sang, en particulier machine de dialyse, avec une pompe péristaltique (2) destinée à transporter un fluide d'un côté basse pression à un côté haute pression, laquelle pompe péristaltique (2) comporte un rotor (18) pouvant tourner autour d'un axe de rotor (19) et une surface d'appui (21) réalisée en forme d'arc autour de l'axe de rotor (19), dans lequel une conduite de fluide (22) élastiquement déformable peut être positionnée entre le rotor (18) et la surface d'appui (21) et est déformée lors de la rotation du rotor (18) en formant un rétrécissement de section transversale entre celui-ci et la surface d'appui (21) de telle sorte que, lorsque le rotor (18) est en rotation par rapport à la surface d'appui (21), un fluide est transporté dans la conduite de fluide (22) du côté basse pression au côté haute pression,
lequel dispositif comporte une pièce de boîtier de machine (100) réalisée comme une pièce formée en tôle (101),
**caractérisé en ce que** la surface d'appui (21) est réalisée par formage dans la pièce de boîtier de machine (100, 101).

2. Dispositif de traitement extracorporel du sang selon la revendication 1, dans lequel la surface d'appui (21) est réalisée par formage à froid, en particulier par emboutissage profond.

3. Dispositif de traitement extracorporel du sang selon la revendication 1 ou 2, dans lequel la surface d'appui (21) est réalisée au bord d'un creux (26) ou d'une bosse pratiqué(e) dans la pièce de boîtier de machine (100, 101).

4. Dispositif de traitement extracorporel du sang selon l'une quelconque des revendications précédentes, dans lequel la surface d'appui (21) est réalisée en forme de cylindre partiel.

5. Dispositif de traitement extracorporel du sang selon l'une quelconque des revendications précédentes, dans lequel la surface d'appui (21) est réalisée inclinée d'un angle α par rapport au plan de tôle de la pièce de boîtier de machine (100, 101), lequel angle α est compris entre 120° environ et 95° environ, de préférence entre 115° environ et 100° environ, de manière de loin préférée entre 110° environ et 105° environ.

6. Dispositif de traitement extracorporel du sang selon l'une quelconque des revendications précédentes, dans lequel la surface d'appui (21) entoure une portion de fond ou une surface de fond (29) qui est réalisée de façon radiale à l'intérieur de la surface d'appui (21), en particulier qui est formée conjointement à la surface d'appui (21).

7. Dispositif de traitement extracorporel du sang selon la revendication 6, dans lequel la portion de fond ou la surface de fond (29) réalise une surface de palier de butée pour la conduite de fluide (22) et/ou le rotor (18).

8. Dispositif de traitement extracorporel du sang selon l'une quelconque des revendications précédentes, dans lequel l'axe de rotor (19) est parallèle à la surface d'appui (21).

9. Dispositif de traitement extracorporel du sang selon l'une quelconque des revendications 3 à 8, dans lequel le creux (26) comporte un contour extérieur sensiblement en forme de fer à cheval, dans lequel des surfaces d'entrée respectivement de sortie (27, 28) qui sont réalisées de préférence parallèles à l'axe de rotor (19) sont réalisées des deux côtés de la surface d'appui (21).

10. Pièce de boîtier (100, 101) pour un dispositif de traitement extracorporel du sang, en particulier une machine de dialyse, en particulier selon l'une quelconque des revendications précédentes, dans lequel la pièce de boîtier (100, 101) est formée à partir d'une tôle,
**caractérisée en ce**
**qu'**un creux (26) est réalisé par formage dans la pièce de boîtier (100, 101), lequel creux sert à loger un rotor (18) pouvant tourner autour d'un axe de rotor (19) et une portion de conduite de fluide (22) élastiquement déformable d'une pompe péristaltique (2), dans laquelle un bord, réalisé en forme d'arc autour de l'axe de rotor (19), du creux constitue une surface d'appui (21) contre laquelle peut être poussée la portion de conduite de fluide (22) au moyen du rotor en vue du rétrécissement de section transversale.

11. Procédé de fabrication d'une pièce de boîtier (100, 101) pour un dispositif de traitement extracorporel du sang, en particulier une machine de dialyse, en particulier selon l'une quelconque des revendications 1 à 10, avec les étapes :
formage de la pièce de boîtier (100, 101) à partir d'une tôle ; et
réalisation d'un creux (26) dans la pièce de boîtier (100, 101) par formage, lequel creux sert à loger un rotor (18) pouvant tourner autour d'un axe de rotor (19) et une portion de conduite de fluide (22) élastiquement déformable d'une pompe péristaltique (2), dans lequel un bord, réalisé en forme d'arc autour de l'axe de rotor (19), du creux constitue une surface d'appui (21) contre laquelle peut être poussée la portion de conduite de fluide (22) au moyen du rotor en vue du rétrécissement de section transversale.
